Europäisches Patentamt

European Patent Office

Office européen des brevets

(19)

(11) Publication number: **0 319 711**

**A1**

(12)

# EUROPEAN PATENT APPLICATION

(21) Application number: 88118340.4

(51) Int. Cl.⁴: **A61N 1/04**

(22) Date of filing: 04.11.88

(30) Priority: 07.12.87 JP 185462/87
29.08.88 JP 112971/88

(43) Date of publication of application:
14.06.89 Bulletin 89/24

(84) Designated Contracting States:
DE FR GB IT

(71) Applicant: **FUKUDA DENSHI CO., LTD.**
**39-4, Hongo 3-chome Bunkyo-ku**
**Tokyo 113(JP)**

(72) Inventor: **Shimizu, Chuji Fukuda Denshi Co.,**
**Ltd.**
**Hongo Enterprise Place 2-35-8 Hongo**
**Bunkyo-ku Tokyo(JP)**

(74) Representative: **Behn, Klaus, Dipl.-Ing.**
**Patentanwalt Lindenberg 34**
**D-8134 Pöcking bei München(DE)**

(54) **Pad for using for the living body stimulus electrode.**

(57) A stimulus electrode, comprising two pads respectively composed of two conductive and adhesive gel hydrate layers which pinch on the sides of an insulated substrate sheet, and which are connected to each other, said two pads corresponding to a first and second electrode plate and being integrated within the body of a low-frequency small version medical treatment apparatus, an exposed center portion of said sheet between the pads, a grip projecting away from the pads, or
and a bonding layer disposed at least partially on one side surface of a surrounding edge portion and center portion of said sheet, said bonding layer being capable of being bonded to said treatment apparatus.

Fig. 3

## PAD FOR USING FOR THE LIVING BODY STIMULUS ELECTRODE

### BACKGROUND OF THE INVENTION

#### 1. Field of the Invention

The present invention relates to pad for using for the living body stimulus electrode. More particularly, it relates to pad applicable to the living body stimulus electrodes integrated with the low frequency medical treatment apparatus in small style.

#### 2. Description of the Related Art

Generally, a living body electrode is defined a lead element for leading electric phenomenon or electric stimulus by contacting with the living body, which living body electrode is devided into two main classes as follows.

That is to say, the living body electrode is devided into a living body lead relectrode for leading out electric phenomenon without the living body and a living body stimulus electrode for leading in electric stimulus within the living body.

For example, the living body lead electrode is used when electric potentials between two points are led out from the surface of the living body, inputting in the electrocardiograph, and thereby, the gelectrocardiogram is output. On the other hand, the living body stimulus electrode is used when the pulse electric current is led into the living body from the low frequency medical apparatus, and thus, muscle stiff or neuralgia is eased.

However, it is undesirable to contact above both electrodes directly with the living body, which reason is as follows.

1 Since an electrode plate by which the electrode is constructed, is metallic, the electrode plate cannot contact with easily the living body.

2 Owing to the contact resistance between the skin surface of the living body and the electrode plate, it is difficult to lead exactly electric phenomenon or electric stimulus.

3 The electrode is used by many patients, and thereby, dirt attached to their skin or disease germ adheres to the electrode. The result is that since the electrode becomes to be injurious to their health, it is an issue of importance that they are infected each other.

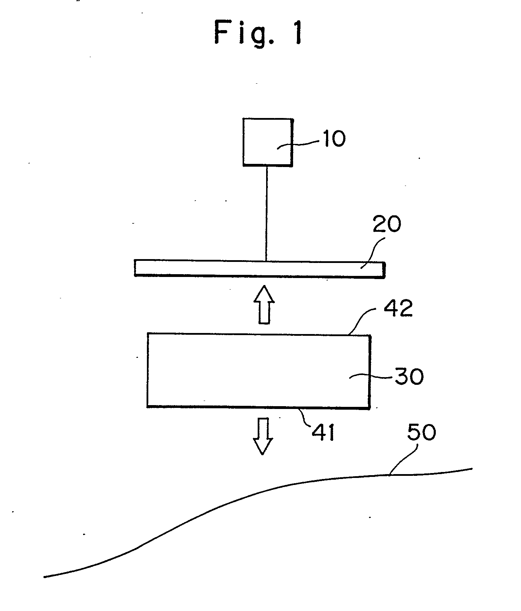

Therefore, the living body lead and stimulus electrodes are both is constructed by the electrode plate 20 connected to a medical apparatus 10 of the electrocardiograph or low frequency medical treatment apparatus as well as by a pad 30 connective with the electrode plate 20, as shown in Fig. 1.

The pad 30 is composed of layer made from conductive and adhesive gelhydrate, and when it is used, one face 42 thereof is sticked to the electrode plate 20, the other face 41 thereof contacted closely with the skin surface 50 of the living body.

That is to say, the living body electrode is constituted by the pad 30 made of gelhydrate layer combining conductivity and adhesion, except for the plate 20. Moreover, the pad 30 is capable of bonded to or taken off the plate 20.

Accordingly, the defects stated in the items 1 to 3 are solved simultaneousely.

In the pad 30 with such a meaning, the present invention relates to, in particular, a pad for the living body stimulus electrode applicable to the low frequency medical treatment apparatus in small style.

Figure 2a is an explanatory drawing of the first prior art.

A low frequency medical treatment apparatus in accordance with the first prior art has a first electrode plate 5 and a second electrode plate 6, which are mounted to the top of pad cords 8 and 9 connected with a body 7 and are separate each.

A first pad 1 and a second pad 2 are respectively capable of bonded to or taken off the plates 5 and 6.

When the low frequency medical treatment apparatus is used, the first and second pads 1 and 2 are respectively sticked to the first and second electrode plates 5 and 6, through a first recess 5 A and a second recess 6 A, and then, the surface of the pads 1 and 2 are contacted closely with, for example, the skin surface of both shoulders of the patient.

However, as electronic parts become smaller, the low frequency medical treatmest apparatus composed of them also does.

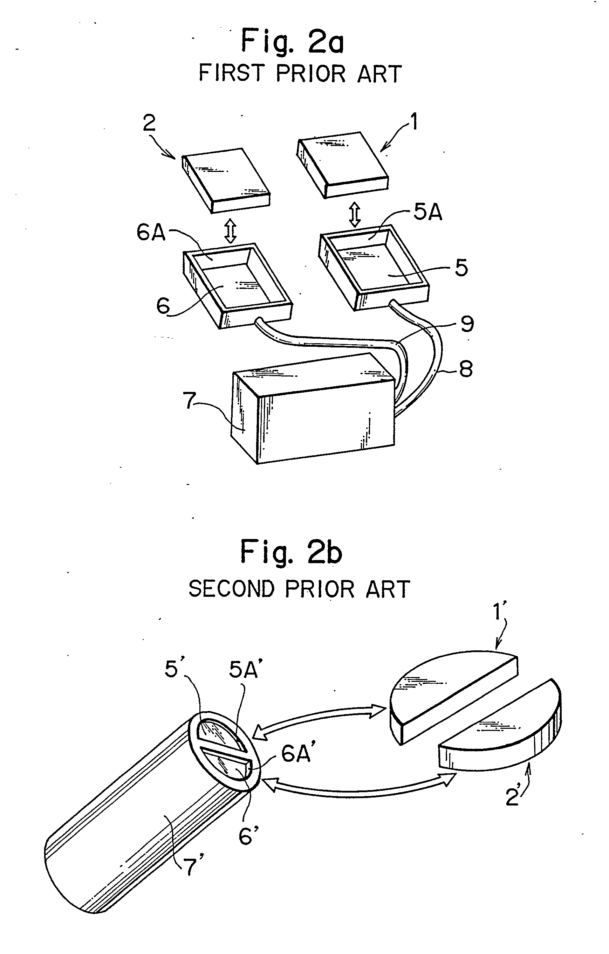

The low frequency medieal treatment apparatus in small style, so-called "handy type" has recently been proposed, as shown in Fig.2b.

In the second prior art of Fig. 2b, a first electrode plate 5′ and a second electrode plate 6′ are mounted on a body 7′, not through the pad cords 8 and 9(see Fig.2a), but directly.

In other words, the first and second electrode plates 5′ and 6′ are integrated with the body 7′ as well as are adjacent eact other. Corresponding first and second pads 1′ and 2′ are respectively capable of bonded to or taken off the plates 5′ and 6'.

The problems of the second prior art are as follows.

(1) The pads 1' and 2' must be separately bonded to or taken off the electrode plates 5' and 6'.

The result is that the above bonding and taking operations should be executed respectively twice, which is very troublesome for the user.

Moreover, since the size of the pads 1' and 2' are each nearly equal to that of corresponding recesses 5A' and 6A' it is very difficult to put the small pads 1' and 2' in or out the recesses 5A' and 6A' respectively, by gripping directly them with the fingers of the user's hand.

It is more difficult for the user to carry out the above operations, because the fingers and the pads become to attach firmly each other owing to adhesion of gelhydrate layer by which the pads 1' and 2' are constituted.

Consequently, it takes long time to adhere the pads 1 and 2 to the electrode plates 5' and 6' respectively.

(2) Since the small pads are gripped directly with the fingers, the dirt, for example dust etc.,on the fingers adheres to the whole gelhydrate, and thereby, the pads lose conductivity and adhesion thereof.

In particular, according to losing adhesion, the defect of the second prior art is as follows.

That is to say, after medical treatment, when the pads 1' and 2' are taken off from the skin surface of the living body together with the body 7' , the pads fall off from the body 7' and keep to contact closely with the skin surface, due to which the patient feels unpleasantness.

The reason is that when the pads lose adhesion thereof, they become attached to the skin surface of the living body more than the electrode plate made of metal.

In a word, the secoud prior art has the problems as follows.

That is to say, even if the pads are intended to be bonded to or taken off the corresponding electrode plates of the low frequeneey medieal treatment apparatus in amall style, since the pads are small and separate each other, it takes long time to execute the above bonding and taking operations.

Moreover, since the pads do not become attached firmly to the electrode plate, they fall off easily from the above apparatus in small style.

## SUMMARY OF THE INVENTION

An object of the present invention is to execute, with ease and rapidity, the bonding and taking operations of the pads for the living body stimulus electrode regarding to the low frequencey medical treatment apparatus.

Another of the present invention is, adding to the above object, to prevent those pads from falling off from the same apparatus.

The above-mentioned first object can be achieved by pad for using for the living body stimulus electrode, comprising, a first pad composed of a first living body side gelhydrate layer and a first electrode side gelhydrate layer, having repectively conductivity and adhesion, which pinch one side portion of an insulated substrate sheet and are connected each other, and a second pad composed of a second living body side gelhydrate layer and a second electrode side gelhydrate layer, having respectively conductivity and adhesion, which pinch the other side portion of said insulated substrate sheet and are connected each other, said first and second pads corresponding respectively to the first and second electrode plates and adjacent integrated with the body of the low frequency medical treatment apparatus in small style, a center portion of said sheet exposed continuously and wholly, said first and second pads, a grip projecting towards the outside of said first and second pads.

The above-mentioned second object can be achieved by pad for using for the living body stimulus electrode, comprising, a first pad composed of a first living body side gelhydrate layer and a first electrode side gelhydrate layer, having repectively conductivity and adhesion, which pinch one side portion of an insulated substrate sheet and are connected each other, and a second pad composed of a second living body side gelhydrate layer and a second electrode side gelhydrate layer, having respectively conductivity and adhesion, which pinch the other side portion of said insulated substrate sheet and are connected each other, said first and second pads corresponding respectively to the first and second electrode plates and adjacent integrated with the body of the low frequency medical treatment apparatus in small style, a center portion of said sheet exposed continuously and wholly, between said first and second pads, a bonding layer disposed at least partially on one side surface of a surrounding edge portion of said sheet, which portion projects towards said center portion as well as said first and second pads, said bonding layer capable of bonding to said body.

## BRIEF DESCRIPTION OF THE DRAWINGS

Further features and advantages of the present invention will be apparent from the ensuring description with reference to the accompanying drawings, wherein:

Fig. 1 is a general explanatory drawing of the living body electrode;

Fig. 2a is an explanatory drawing of a first prior art;

Fig. 2b is an explanatory drawing of a second prior art;

Fig. 3 is a drawing of the principle of the first present invention;

Fig. 4a is a perspective drawing of the first embodiment of the first present invention;

Fig. 4b is a drawing of the relationship between the living body side gelhydrate layer and the electrode side gelhydrate layer of the first embodiment of the first present invention;

Fig. 4c is a drawing of the grip of the first embodiment of the first present invention;

Fig. 5a is a perspective drawing of the second embodiment of the first present invention;

Fig. 5b is a drawing of the relationship between the living body side gelhydrate layer and the electrode side gelhydrate layer of the second embodiment of the first present invention;

Fig. 6 is a explanatory drawing of the operation of the first present invention;

Fig. 7 is a drawing of method for producing of an embodiment of the first present invention;

Fig. 8 is a drawing of the principle of the second present invention;

Fig. 9a is a perspective drawing of the front surface of the first embodiment of the second present invention;

Fig. 9b is a drawing of the relationship between the living body side gelhydrate layer and the electrode side gelhydrate layer of the first embodiment of the second present invention;

Fig. 9c is a perspective drawing of the back surface of the first embodiment of the second present invention;

Fig. 10 is a perspective drawing of the second embodiment of the second present invention;

Fig. 11 is a explanatory drawing of the operation of the second present invention; and,

Fig. 12 is a drawing of method for producing of a embodiment of the second present invention.

DESCRIPTION OF THE PREFERRED EMBODIMENTS

(1) First present invention

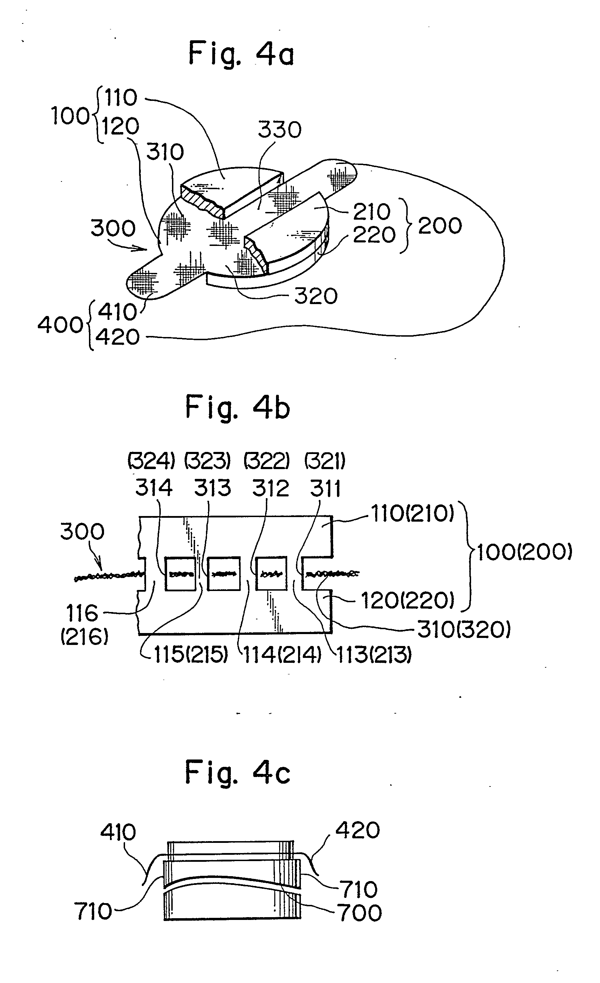

Figures 4a, 4b and 4c are drawings of the first embodiment of the first present invention.

In Fig. 4a, reference numeral 100 shows a first pad, 200 a second pad, 300 an insulated substrate sheet

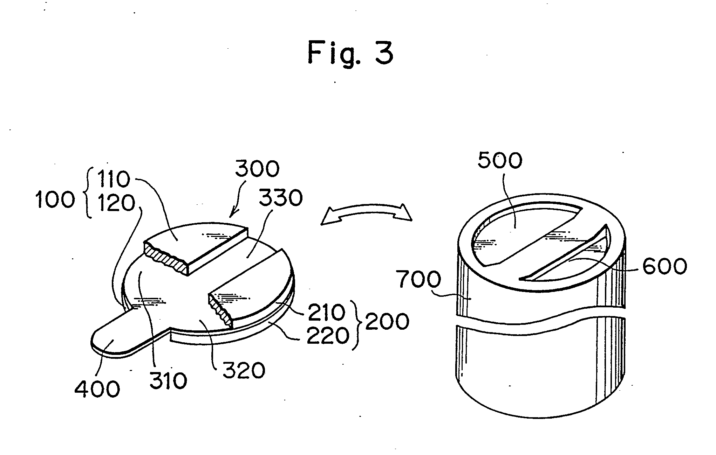

The first and second pads 100 and 200 respectively correspond to a first and second electrode plates 500 and 600 (see Fig. 3), which plates are integrated with the body 700 of the low frequency medical treatment apparatus in small atyle and are adjacent each other.

Since the electrode plates 500 and 600 are respectively formed as semidisks in the first embodiment, the pads 100 and 200 are each formed as low semicolums.

The insulated substrate sheet 300 is made of perforated member, for example texitile, knit, nonwoven fabric etc., and is formed as disk.

The first pad 100 is composed of a first living body side gelhydrate layer 110 and a first electrode side gelhydrate layer 120.

The layers 110 and 120 are respectively conductive and adhesive, and they pinch one side portion 310 of the sheet 300.

Accordingly, the first pad 100 has two layers structure.

The first living body side gelhydrate later 110 is capable of bonded to the skin surface of the living body, and the first electrode side gelhydrate layer 120 does to the surface of the electrode plate.

Both layers 110 and 120 are connected each other by first gelhydrate columns 113, 114 (see Fig. 4b), which columns run through holes 311, 312 of one side portion 310 of the sheet 300.

That is to say, the layers 110 and 120 are connected electrically

The second pad 200 has also the same constitution as the first pad 100, as apparently from Figs, 4a and 4b, and only differs from the first pad 100 in pinching the other side portion 320.

A center portion 330 of the sheet 300 is exposed continuously and wholly, between these both pads 100 and 200.

That is to say, since both pads 100 and 200 are opposed each other through the exposed center portion 330, they are linked while insulated completely.

Both ends of the exposed center portion 330 project towards the outside of the pads 100 and 200, which ends are respectively a first grip 400 and a second grip 420.

Thus, a grip 400 is formed by the first and second grips 410 and 420.

When the pads 100 and 200 are setted to the body 700 of the low frequency medical treatment apparatus in small style, the grips 410 and 420 are bent, and thereby they are capable of bonded to the outside 710 of the body 700.

According, there is the effect that the pads 100 and 200 contact closely with the skin surface of the lining body, together with the body 700.

In other words, the pads 100 and 200 are prevented from falling off the body 700.

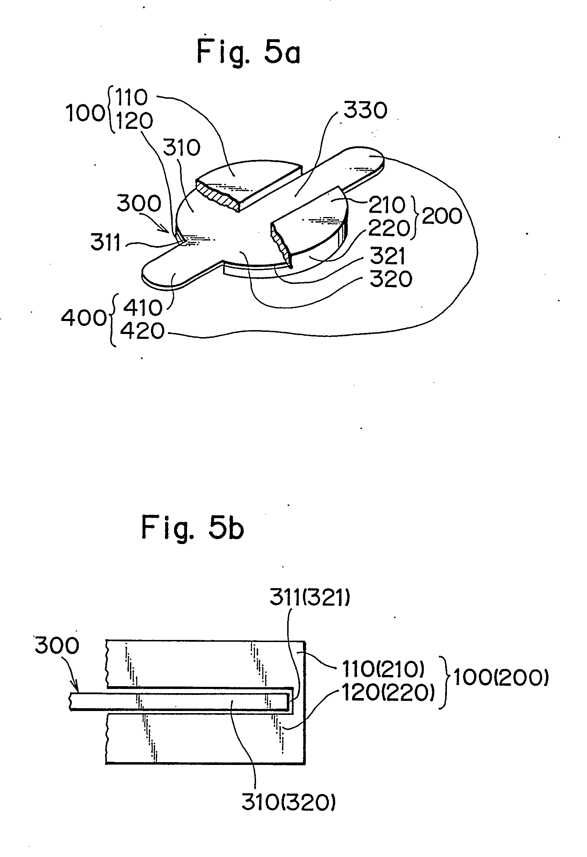

Figs. 5a and 5b are drawings of the second embodiment of the first present invention

The second embodiment differs from the first embodiment (see Figs. 4a, 4b and 4c), in member constituting the insulated substrate sheet 300 and in means for connecting electrically the living body side gelhydrate layer with the electrode side gelhydrate layer.

That is to say, in the second embodimet, the sheet 300 is made of none-perforated member, for example rubber, plastic etc.

Accordingly, the layers 110 and 120 are connected each other electrically along the end face 311 of one side portion 310 in the sheet 300.

With sespect to the layers 210 and 220, they have also the same means for connecting as the layers 110 and 120 ( see Figs 5a and 5b ).

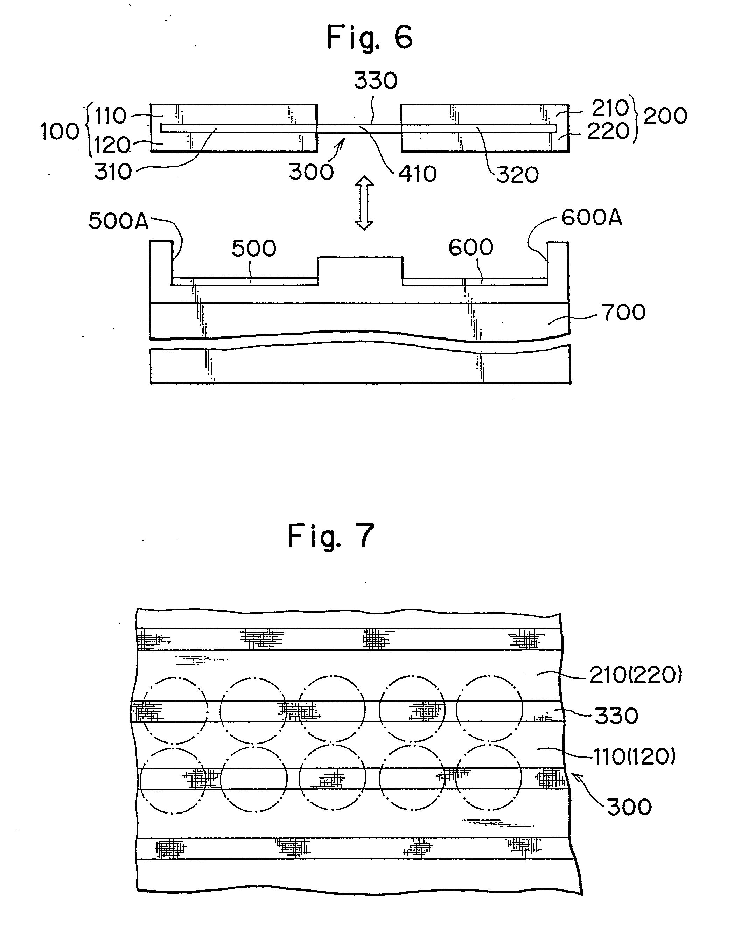

The operation will be described hereinafter with reference to Fig. 6.

At first , after the layers 120 and 220 are turned downwards by pinching the grip 410 with two fingers of the hand, they are made to be bonded respectively to the electrode plates 500 and 600 in the recesses 500A and 600A of the body 700.

Thereby, both pads 100 and 200, out of direct touch with the fingers and similnaneously,are capable of bonded to both electrode plates 500 and 600.

Moreover, the grips 410 and 420 are bent, which are bonded to the outside 710 of the body 700 (see Fin. 4c).

After that, the layers 110 and 210 exposed out of the recesses 500A and 600A are made to contact closely with the skin surface of the hand or leg etc., by holding the body 700 with the pads 100 and 200.

Thereby, since the pads and the body 700 contacts closely together with the living body, they do not fall off during medical treatment.

After treatment, the layers 110 and 210 are taken off, together with the body 700, from the skin surface and the grips 410 and 420 are returned, being taken off from the outside 710.

The layers 120 and 220 are taken off from the correspouding electrode plates 500 and 600, by pinching again the grip 410 with two fingers of the hand.

Thereby, both pads 100 and 200 are also, simultaneously once and without direct touch with the fingers, capable of taken off from the electrode plates 500 and 600.

Figure 7 is a drowing of method for producing of an embodiment of the first present invention.

When an solution, composed of conductive and adhesive acryl gelhydrate, is made to be penetrated from the upper surface of the sheet 300 made of knit member through holes thereof, it becomes to penetrate at the down surface of the sheet 300.

Thereby, when the solution becomes solid by applying ultraviolet rays, the living body side and electrode side gelhydrate layers are formed so as to pinch the sheet 300,

Therefore, two gelhydrate layers, which pinches the sheet 300, are capable of disposed in many rows on the opposite sides of the portion where the sheet 300 is exposed as shown in Fig. 7.

Accordiugly, many pads of the first present invention are produced by cutting, as shown by chain line of Fig. 7.

(2) The second present invention

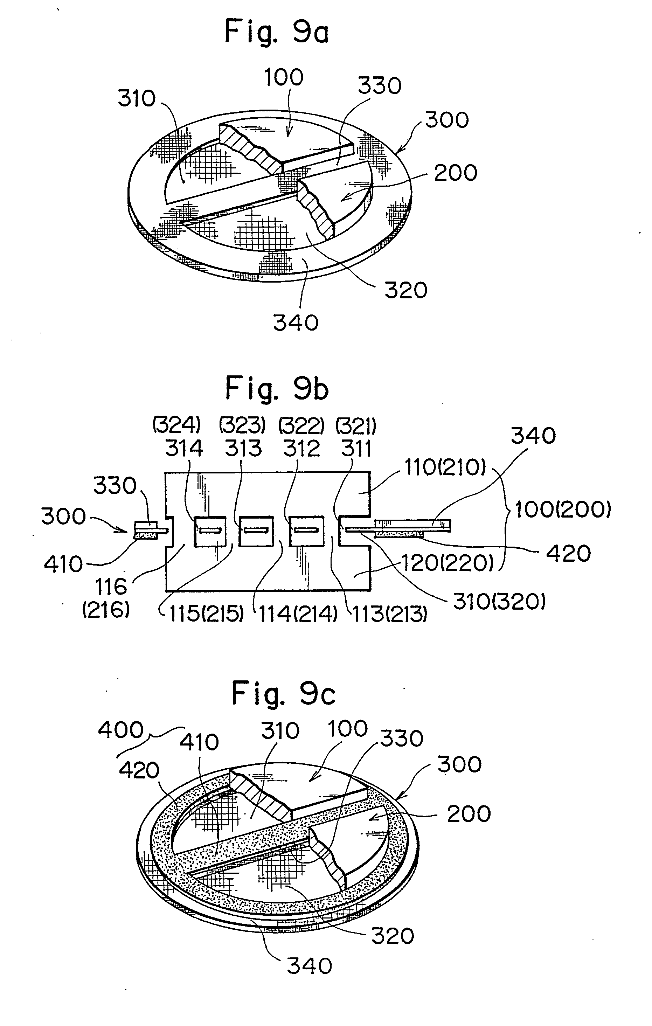

Figures 9a, 9b and 9c are drawings of the first embodiment of the second present invention.

In Fig. 9a, reference numeral 100 shows a first pad, 200 a second pad, 300 an insulated substrate sheet

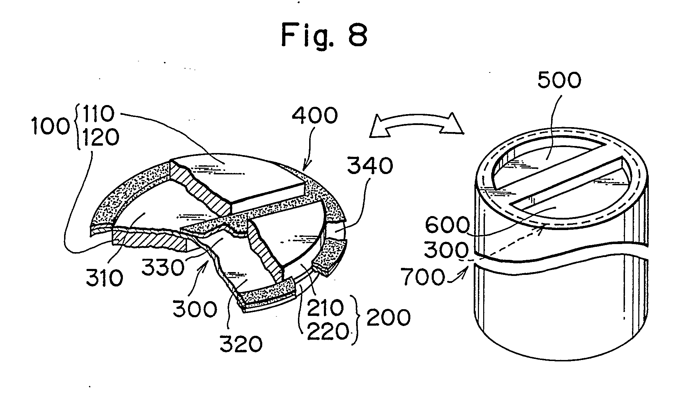

The first and second pads 100 and 200 respectively correspond to a first and second electrode plates 500 and 600 (see Fig. 8), which plates are integrated with the body 700 of the low frequency medical treatment apparatus in small style and are adjacent each other.

Since the electrode plates 500 and 600 are respectively formed as semidisks in the first embodiment, the pads 100 and 200 are also each formed as low semicolums.

One and the other side portions 310 and 320 of the sheet 300 are made of absorbent member, for example bemberg nonwoven fabric.

A center portion 330 of the seet 300 is formed by putting repellent member, for example a mixture of self linking acryl and titanium oxide, upon the bemberg nonwoven fabric see Fig. 9a

A surrounding edge portion 340 of the sheet 300 is formed by putting a bonding layer 400, for example acryl bonding agent, upon the back surface of the bemberg nonwoven fabric ( see Fig. 9c ).

The bonding layer 400 may be disposed wholly on the back surfaces of the center and surrounding edge portions 330 and 340.

Alternatively, the bonding layer 400 may be arranged on the whole portion 410 and on the partial portion 420 positioning respectively in the back surfaces of the center and surrounding edge portions 330 and 340, as shown in Fig. 9c .

The first pad 100 is composed of a first living body side gelhydrate layer 110 and a first electrode side gelhydrate layer 120.

The layers 110 and 120 are respectinely conductive and adhesive, and they pinch one side portion 310 of the sheet 300.

Accordingly, the first pad 100 has two layers structure.

The first living body side gelhydrate later 110 is capable of bonded to the skin surface of the living body, and the first electrode side gelhydrate layer 120 does to the surface of the electrode plate.

Both layers 110 and 120 are connected each other by first gelhydrate columns 113, 114 • • • - (see Fig. 9b), which columns run through holes 311, 312 • • • of one side portion 310 of the sheet 300.

That is to say, the layers 110 and 120 are connected electrically .

The second pad 200 has also the same constitution as the first pad 100, as apparently from Figs, 9a and 9b, and only differs from the first pad 100 in pinching the other side portion 320.

A center portion 330 of the sheet 300 is exposed continuously and wholly, between these both pads 100 and 200.

That is to say, since both pads 100 and 200 are opposed each other to the exposed center portion 330, they are linked while insulated completely.

Moreover, the surrounding edge portion 340 projects towards the outside of the pads 100 and 200 so as to surround them.

If the pads 100 and 200 be mounted on the body 700 by gripping the surrounding edge portion 340 with fingers, they will, as mentioned later, becomes not to fall off the body 700 owing to the operation of the bonding layer 400 .

In other words, thre is the effect to prevent the pads from falling off the low frequency medical treatment apporatus in small style.

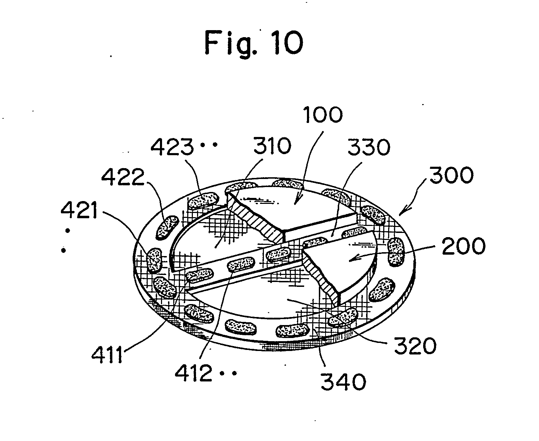

Figure 10 is a drawing of the second embodiment of the second present invention.

The second embodiment differs from the first embodiment ( see Figs. 9a, 9b and 9c ) in mode of disposition of the bonding layer on the back surfaces of the center and surrounding edge portions 330 and 340.

That is to say, the bonding layer of the second embodiment is disposed partially on the center and surrounding edge portions 330 and 340, which layer consists of a plurarity of bonding layers 411, 412 • • • 421, 422 • • • placed at regular intervals.

Since the other elements of the second embodiment are completely equal to those of the first embodiment, explanatiin thereof will be omitted.

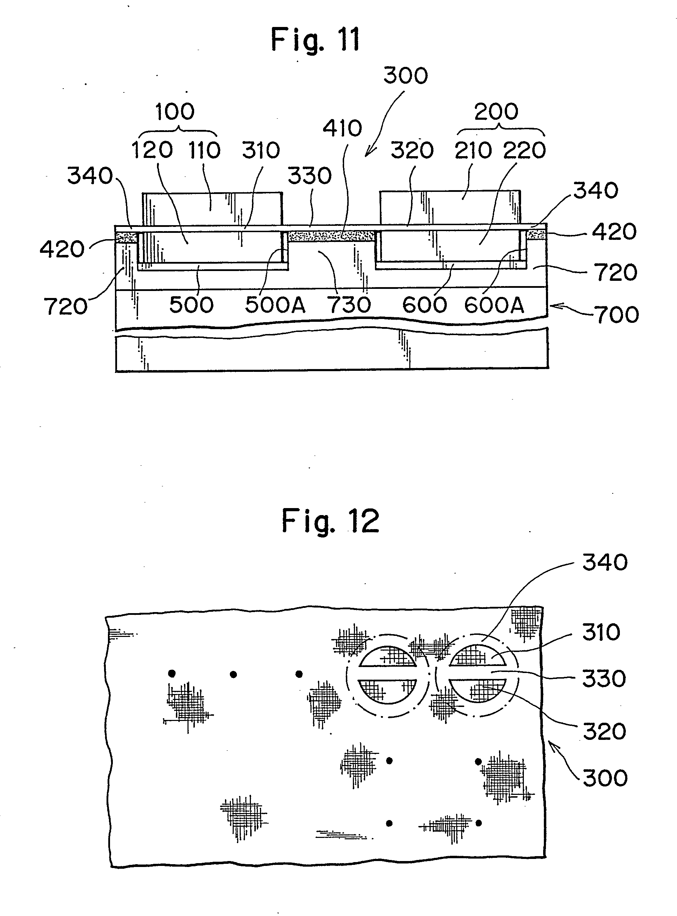

The operation of the second present invention will be described hereinafter with reference to Fig. 11.

At first , after the layers 120 and 220 are turned downwards by pinching the surrounding edge portion 340 with two fingers of the hand, they are made to be bonded respectively to the electrode plates 500 and 600 in the recesses 500A and 600A of the body 700, and the bonding layers 410 and 420 do to the corresponding center and surrounding edge portions 730 and 720 of the body 700.

Thereby, both pads 100 and 200, out of direct touch with the fingers and similtaneously, are capable of bonded to both electrode plates 500 and 600 as well as the center and surrounding edge portions 730 and 720.

After that, the layers 110 and 210 exposed out of the recesses 500A and 600A are made to contact closely with the skin surface of the hand or leg etc., by holding the body 700 with the pads 100 and 200.

Thereby, both pads and the body 700 contacts closely together with the living body.

After treatment, the layers 110 and 210 are taken off together with the body 700, from the skin surface. In this case, since the bonding layers 410 and 420, according to the second present invention, is desposed, the pads 100 and 200 do not fall off from the body 700.

The gelhydrate layers 120 and 220, the bonding layer 410, and the bonding layer 420 are taken off respectively, from the correspouding electrode plates 500 and 600, the corresponding center portion 730, and the corresponding surrounding edge portion 720, by pinching again the surrounding edge portion 340 with two fingers of the hand.

Thereby, both pads 100 and 200 are, simultaneously once and without direct touch with the fingers, capable of taken off from the electrode plates 500 and 600.

Figure 12 is a drawing of method for producing of an embodiment of the second present invention.

A repellent member, for example a mixture of self linking acryl and titanium oxide, in which openings are formed on the region corresponding to one and the other side portions 310 and 320, is putted upon the upper surface of the sheet 300 made of an absorbent member, for example bemberg nonwoven fabric.

On the other hand, adhesive agent of acryl is applied to the down surface of the sheet 300, except for those openings.

Accordingly, the bonding layers are disposed on the down surface of the center and surrounding edge portions 330 and 340 of the sheet 300.

After that, a solution, composed of conductive and adhesive acryl gelhydrate, is made to be penetrated from the upper surface of the sheet 300 towards those openings.

Since the portions 310 and 320 within those openings are made of absorbent member, the solution runs through only these portions and is repelled from the center and surrounding edge portions 330 and 340 made respectively of repellent member.

Thereby, the solution becomes also to appear on the down surface of the portions 310 and 320.

Concequently, when the solution becomes solid by applying ultraviolet rays, the living body and electrode side gelhydrate layers are formed so as to pinch the sheet 300.

After the above processes, when the sheet 300 is cutted as shown by chain line of Fig. 11, many pads of the second present invention are produced.

According to the first present invention, since the first and second pads are linked each other while insulated completely, when the grip is holded with fingers, these two pads are, simultaneously and out of direct touch with the hand, capable of bonded to and taken from the body of the low frequency medical treatment apparatus in small style.

Thereby, the first present invention has the effect that the pad for using for the living body stimulus electrode is, with ease and rapidity, bonded to and taken off from the body of the low frequency medical treatment apparatus in small style.

According to the second present invention, since the bonding layer 400 is disposed to the pads, adding to the elements of the first present invention, it is capable of firmly bonded to the corresponding center and surrounding edge portions 730 and 720 of the body 700.

Thereby, the second present invention has, adding to the above effect of the first present invention, the effect that the pad for using for the living body stimulus electrode is prevented from falling off the body of the low frequency medical treatment apparatus in small style.

## Claims

1. Pad for using for the living body stimulus electrode comprising:
a first pad (100) composed of a first living body side gelhydrate layer (110) and a first electrode side gelhydrate layer (120), having repectively conductivity and adhesion, which pinch one side portion (310) of an insulated substrate sheet (300) and are connected each other, and
a second pad (200) composed of a second living body side gelhydrate layer (210) and a second electrode side gelhydrate layer (220), having respectively conductivity and adhesion, which pinch the other side portion (320) of said insulated substrate sheet (300) and are connected each other,
said first and second pads (100) and (200) corresponding respectively to the first and second electrode plates (500) and (600) adjacent integrated with the body (700) of the low frequency medical treatment apparatus in small style,
a center portion (330) of said sheet (300) exposed continuously and wholly, between said first and second pads (100) and (200),
a grip (400) projecting towards the outside of said first and second pads (100) and (200).

2. Pad for using for the living body stimulus electrode according to claim 1,
wherein said grip (400) comprises a first grip (410) and a second grip (420), which are respectively flexible and capable of bonding to said body (700).

3. Pad for using for the living body stimulus electrode comprising:
a first pad (100) composed of a first living body side gelhydrate layer (110) and a first electrode side gelhydrate layer (120), having repectively conductivity and adhesion, which pinch one side portion (310) of an insulated substrate sheet (300) and are connected each other, and
a second pad (200) composed of a second living body side gelhydrate layer (210) and a second electrode side gelhydrate layer (220), having respectively conductivity and adhesion, which pinch the other side portion (320) of said insulated substrate sheet (300) and are connected each other,
said first and second pads (100) and (200) corresponding respectively to the first and second electrode plates (500) and (600) adjacent integrated with the body (700) of the low frequency medical treatment apparatus in small style,
a center portion (330) of said sheet (300) exposed continuously and wholly, between said first and second pads (100) and (200),
a bonding layer (400) disposed at least partially on one side surface of a surrounding edge portion (340) of said sheet (300), which portion (340) projects towards the outside of said center portion (330) as well as said first and second pads (100) and (200),
said bonding layer (400) capable of bonding to said body (700).

4. Pad for useing for the living body stimulus electrode according to claim 3,
wherein said one and the other portions (310) and (320) of said sheet (300) are made of absorbent member,
said center portion (330) of said sheet (300) is formed by putting repellent member upon the front surface of said absorbent member, and
said surrounding edge portion (340) of said sheet (300) is formed by putting said bonding layer (400) upon the back surface of said absorbent member.

5. Pad for using for the lving body stimulus electrode according to claim 3,
wherein said bonding layer (400) is disposed wholly on said center and surrounding edge portions (330) and (340) of said steet(300).

6. Pad for using for the living body stimulus electrode according to claim 3, wherein said bonding layer (400) is dcsposed wholly on said center portion (330) and partially on said surrounding edge portion (340).

7. Pad for using for the living body stimulus electrode accrding to claim 3, wherein said bonding layer (400) is disposed partially on said center and surrounding edge portions (330) and (340).

# Fig. 1

# Fig. 2a
## FIRST PRIOR ART

# Fig. 2b
## SECOND PRIOR ART

# Fig. 3

# Fig. 4a

100 { 110
      120

310

330

300

210 } 200
220

320

400 { 410
      420

# Fig. 4b

(324) (323) (322) (321)
314   313   312   311

300

116
(216)

110(210)

100(200)

120(220)

310(320)

115(215)  114(214)  113(213)

# Fig. 4c

410

420

710

710

700

710

# Fig. 5a

# Fig. 5b

# Fig. 6

# Fig. 7

## Fig. 8

## Fig. 9a

## Fig. 9b

## Fig. 9c

## Fig. 10

## Fig. 11

## Fig. 12

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 424 165 (J.I. MOSS)<br>* Whole document *<br>--- | 1,3 | A 61 N 1/04 |
| A | US-A-4 383 529 (H.L. WEBSTER)<br>* Column 3, line 4 - column 4, line 48;<br>figures 1-4 *<br>--- | 1,3 | |
| A | DE-A-3 136 366 (NITTO ELECTRICAL)<br>* Page 10, lines 1-11; figure 4 *<br>--- | 1,3 | |
| A | EP-A-0 226 568 (HEPAX LTD)<br>* Column 4, lines 1-49; figures 1,2 *<br>----- | 1,3 | |

TECHNICAL FIELDS
SEARCHED (Int. Cl.4)

A 61 N
A 61 B

The present search report has been drawn up for all claims

| Place of search | Date of completion of the search | Examiner |
|---|---|---|
| THE HAGUE | 08-03-1989 | SCHMIERER U.J. |